# EUROPEAN PATENT APPLICATION

(11) **EP 1 916 543 A1**
(43) Date of publication of application: **30.04.2008**
(21) Application number: 06122804.5
(22) Date of filing: 24.10.2006
(51) Int. Cl.: G01T 1/29

(54) **Triple-modality imaging system**

(71) Applicant: DKFZ Deutsches Krebsforschungszentrum, Stiftung des Öffentlichen Rechts, 69120 Heidelberg (DE)
(72) Inventor: Peter, Jörg, 69198 Schriesheim (DE)
(74) Representative: Huhn, Michael

(57) **Abstract**

The invention relates to a triple-modality imaging system, wherein an optical imaging detector (1) for acquiring optical imaging data, a single photon emission computered tomography (SPECT) detector (3) for acquiring SPECT data, and an x-ray source (5) and detector (6) for acquiring x-ray data are arranged to acquire optical imaging data, SPECT data, and x-ray data of an imaged object simultaneously, the optical imaging detector (1), the SPECT detector (3), and the x-ray detector (6) having spatially over-lapping fields-of view (FOV) (20, 18, 19).

## Description

### Field of the invention

The present invention relates to a triple-modality non-invasive in vivo imaging system and a method for triple-modality imaging using an optical imaging detector for acquiring optical imaging data, a single photon emission computed tomography (SPECT) detector for acquiring SPECT data and an x-ray detector for acquiring x-ray data.

### Background of the invention

A qualitative and quantitative acquisition of morphological, functional and biochemical parameters using imaging methods is the basis for a plurality of medical research and application areas. Three known imaging methods are optical imaging techniques using, e.g. fluorescence or bioluminescence, single photon emission computed tomography (SPECT) using radionuclides and computed tomography (CT) using x-rays. These imaging techniques are all applicable for in-vivo examination.

Optical imaging techniques include fluorescence and bioluminescence imaging. Fluorescence is the result of a process that occurs in certain molecules called fluorophores or fluorescent dyes. A fluorescent probe is a fluorophore designed to localize within a specific region of a biological specimen or to respond to a specific stimulus. In order to perform fluorescence imaging, a photon of certain energy is supplied by an external source such as an incandescent lamp or a laser and absorbed by the fluorophore, creating an excited electronic singlet state. This process distinguishes fluorescence from bioluminescence. It follows that another photon of lower energy is emitted, returning the fluorophore to its ground state. Using an appropriate sensor device, the emitted photon can be detected. Bioluminescence refers to the visible light emission in living organisms that accompanies the oxidation of organic compounds (luciferins) mediated by an enzyme catalyst (luciferase). Unlike fluorescence approaches, the imaged object does not need to be exposed to the light of an external light source. Bioluminescence imaging is carried out by tagging cells with a luciferase gene. These genetically engineered, light emitting cells can be followed throughout the imaged object by means of an appropriate sensor device.

Optical imaging has evolved into a potentially valuable tool for assessing functional and molecular properties, particularly in small animals such as mice and rats. Examples include protein-protein interactions with cells, gene regulation at the transcription level, protein degradation over time, enzymatic activity associated with tumor progression, and cell death. Examples of ongoing applications include cancer, inflammatory disease, neurodegenerative disease, gastrointestinal physiology, renal physiology, cell trafficking, stem cell research, transplant science, and muscle physiology.

Optical planar imaging and optical tomography (OT) are alternative imaging modalities, that detect light propagated through tissue at single (planar) or multiple projections (tomography). Optical imaging applications in vivo will require high efficient photon collection systems. Primary interest for using an optical imaging technique lies in the non-invasive and non-hazardous nature of optical photons used, its low cost, its straightforward technology and most significantly in the availability of activatable probes that produce a signal only when they interact with their targets - as compared to radio labelled probes used in PET (positron emission tomography) and SPECT (single photon emission computed tomography), which produce a signal continuously, independent of interacting with their targets, through the decay of the radioisotope. In OT, images are influenced greatly by the spatially dependent absorption and scattering properties of tissue. Boundery measurements from one or several sources and detectors are used to recover the unknown parameters from a transport model described, for instance, by a partial differential equation. The contrast between the properties of diseased and healthy tissue can be used in clinical diagnosis.

The majority of existing optical imaging approaches are using CCD cameras. CCDs (charge coupled devices) are imaging sensors that serve for sensitive detection of photons. In order to define an optical field-of-view, the CCD detector is typically coupled to a lens. Alternatives for the use if CCDs are avalanche photo diodes (APDs), complementary metal oxide semiconductors (CMOS), or other realizations of photosensitive detectors.

Another imaging method known in the state of the art is single photon emission computed tomography. For SPECT radioactive markers are injected into the animal or human to be examined, which concentrate in specific organs or tissue types of the animal/human depending on the biological carriers which transport them. The radioactive markers emit radioactive radiation (gamma-radiation), the intensity of which in a specific region of a subject to be examined depends on the concentration of the marker in said region. The radioactive radiation is detected by means of a gamma-camera or scintillation camera.

SPECT is a clinical and experimental imaging modality that permits the use of photon-labeled molecular or functional imaging probes for non-invasive assays of biochemical processes. High-energy (keV) photons are particles emitted from a radioactive substance administered to the patient/animal. As in optical imaging, the imaging procedure can be repeatedly performed, thereby allowing each patient/animal to be used as its own control. Photon-labeled compounds have been synthesized for a variety of molecular and functional targets, with examples of biological processes ranging from receptors and synthesis of transmitters in cell communication, to metabolic processes and gene expression. Subsequent transversal views of reconstructed projection data of the imaged object developed by this technique are used to evaluate a variety of diseases. Clinical key objectives of SPECT in oncology, for instance, are to determine and grade tumor mass, to establish whether a tumor is benign or malignant, to locate the site of primary disease, to detect metastatic disease, to identify multi-focal lesions, to determine the extent of tumors for treatment planning, to direct biopsy, to verify prognosis, to monitor response to treatment, to detect local or distant recurrence, and to assess residual mass.

Both beforehand explained imaging modalities provide functional or molecular information often in a complimentary manner. It is common practice to apply both modalities sequentially, using separate devices. While the information acquired is of similar form - although by no means alike - the physical properties of the signal component, the photon, differ strongly. Optical imaging relies on light photons with energies in the order of 2 to 3 eV. On the other hand, isotopes used in SPECT encompass energies in the range of 10 keV to more than 400 keV. As an immediate consequence the interaction processes of both photon classes (optical and isotopic photons) differ significantly (optical photons, for instance, do experience scatter processes several orders of magnitude more often in tissue than isotopic photons) which explains the limited use of optical techniques in large objects such as humans, and also the non-availability of fully three-dimensional image reconstruction algorithms given state-of-the-art.

Based on this, there are several implications for the integration of these two imaging modalities into a single device. One of which is the possible use of information gained from the reconstruction of the isotopic photon distribution for the reconstruction of the optical photon distribution which might ease the solvability of the involved inverse problem. Another implication is to perform optical and SPECT imaging in small animals and - while comparatively investigating and developing both marker classes congruently - carry the SPECT tracer (including its chemistry) further to the clinical applications (i.e. the patient). In short, optical imaging is in most instances not a translational diagnostic technique and cannot, therefore, be applied to both 'mice and man'. However, while adorning the advantages of developing diagnostic procedures and therapy strategies using optical imaging approaches in small animals along with, and primarily instigating, the associated SPECT procedures, the SPECT study can then be potentially carried out on patients. While these principles of dual-modality investigation are primarily of interest for basic drug and tracer development as well as further research activities, more advantages for general laboratory use are explained later.

Another known imaging method is computed tomography using x-rays. In a computed tomography system, an x-ray source is collimated to form a cone or fan beam with a defined beam angle and beam width. The beam is oriented to lie within the X-Y plane of a Cartesian coordinate system, termed the "imaging plane", and to be transmitted through an imaged object to an x-ray detector array oriented within the imaging plane. The detector array is comprised of detector elements, which each measure the intensity of transmitted radiation along a ray projected from the x-ray source to that particular detector element. The intensity of transmitted radiation received by each detector element in the detector array is dependent on the attenuation of the x-ray beam along a ray by the imaged object. Each detector element produces an intensity signal dependent on the intensity of transmitted radiation striking the detector element. The x-ray source and detector array may be rotated on a gantry within the imaging plane so that the beam intercepts the imaged object at different angles. At each angle, a projection is acquired, comprised of the intensity signals of each of the detector elements. The projection at each of these different angles together form a tomographic projection set.

X-ray computed tomography (CT) is an existing powerful non-invasive imaging technique for producing three-dimensional cross-sectional images of an object from two-dimensional x-ray images. CT imaging is based on the absorption of x-rays as they pass through the different parts of the object's volume. Depending on the amount absorbed in a particular tissue such as muscle or lung, a different amount of x-rays will pass through and escape the body. The escaping x-rays interact with a detection device and provide a two-dimensional projection image of the tissues within the object volume. As in OT and SPECT, the use of some kind of mathematical image reconstruction technique yields cross-sectional images of information.

In contrast to SPECT or OT which yield functional or molecular information, x-ray CT delivers anatomical information which is often not present in isotopic or optical photon distributions. At present state-of-the-art (and based on the image generating principle), x-ray CT does not generate functional or molecular information.

In the state of the art these known imaging methods are usually applied separately, using separate detectors successively.

A fusion/registration and comparison of the images obtained by sequentially applied imaging methods is possible only to a limited extent due to organ movement and pharmacokinetics. The problems of excessive and prolonged burdening of the subject to be examined, the non-reproducibility of kinetic studies, the non-identical imaging geometries, animal and organ movement and the correct superposition of the images arise, when two or more methods are carried out successively.

US 2005/0215873 A1 refers to a dual-modality imaging method for simultaneously determining in-vivo distributions of bioluminescent and/or fluorescent markers, and of radioactive markers at identical projection angles. An apparatus for carrying out this imaging method contains at least one CCD-camera, at least one SPECT detector and a layer, which essentially reflects the photons of the bioluminescent and/or fluorescent markers and essentially transmits the photons of the radioactive markers. This apparatus, therefore, relies upon two functional imaging modalities (SPECT and OT). The spatial resolution of the acquired and reconstructed images is in the range of about ≥ 500 µm (at low sensitivity) to 1.4 mm and there is no true anatomical description intrinsically hidden in this data due to the physical characteristics and biochemical properties of both emission tomographic modalities and marker/tracer compounds. For instance, despite the ability of radio-isotope tracers and optical markers to home in on likely areas of primary tumor and malignancy, low spatial resolution can deter precise localization of pathology.

### Summary of the invention

Therefore, the present invention is based on the object of avoiding the disadvantages of the prior art and of improving diagnostic accuracy of SPECT and OT. It is another object of the invention to assess a visual representation, characterization, and possibly even quantification of functional and/or molecular biological processes at cellular and sub-cellular levels aligned with the anatomical structures within an imaged object, preferably within an intact living organism, by means of simultaneously performed image acquisition procedures.

These objects are achieved by means of a triple modality imaging system, wherein an optical imaging detector for acquiring optical imaging data, a single photon emission computed tomography (SPECT) detector for acquiring SPECT-data and an x-ray source and detector for acquiring x-ray data are arranged to acquire the optical imaging data, the SPECT-data and the x-ray data of an imaged object simultaneously, the optical imaging detector, the SPECT detector and the x-ray detector having spatially over-lapping fields-of view (FOV).

The objects are further achieved by a triple-modality imaging method comprising simultaneously acquiring optical imaging data with an optical imaging detector, acquiring single photon emission computed tomography data with a SPECT detector and acquiring x-ray data with an x-ray source and detector of an imaged object which is placed within spatially over-lapping fields-of-view (FOV) of the optical imaging detector, the SPECT detector and the x-ray detector.

In this context the term "simultaneously" comprises the acquiring of the data at the same time and it comprises a negligibly small delay between the acquiring of the data with the different modalities.

Triple-modality OT/SPECT/CT-imaging as proposed by the present invention generates accurately aligned functional/molecular and morphological data sets with a single examination session, thus overcoming the limitations of separate image acquisition. It provides for a single procedural, simultaneous projection data acquisition and image reconstruction of
i) time-resolved in vivo distributions of single- or multi labeled low-energy (light) fluorescence or bioluminescence optical probes (OT), and
ii) high-energy (various radioisotopes) photon emitting (SPECT) molecular markers, and
iii) imaging of the internals from a series of high-resolution x-ray images (CT)
in a small object, particularly in mice and rats, but also in specific human organs and tissues such as breast and skin.

The invention solves problems connected to separately imaging modalities i) to iii) (i.e. sequentially imaging the object with different devices) as for instance the direct study of tracer/marker kinetics, image registration, time-resolved concurrent data analysis and animal handling which are inaccessible (kinetics) or become crucial (registration, animal management). The invention assesses visual representation, characterization, and quantification of anatomical structures and functional or/and molecular biological processes at the cellular and sub-cellular levels within intact living organisms by means of simultaneously performed image acquisition procedures. The invention proposes an instrumentation system that is highly sensitive in identifying location, magnitude, and time variation of specific molecular events (e.g., gene expression and enzyme activity) by simultaneously detecting above listed tracer and marker types in vivo while, with the same acquisition procedure, this spatially low-resolution information can be over-imposed with the spatially high-resolution details of the anatomical structures of the imaged object, and for improvement of attenuation correction of SPECT optical projection data by means of CT-derived three-dimensional anatomical maps, improving diagnostic accuracy of SPECT and OT.

The acquired and reconstructed SPECT and OT images (with the spatial resolution in the range of about ≥ 500 µm) can be aligned with the acquired and reconstructed images of the high-resolution (in the range of about ≥ 100 µm) x-ray imaging (CT). This will, e.g. aid precise tumor localisation.

The present invention further solves problems connected to separately acquiring data with the three different modalities, as for instance
• non-identical imaging geometries,
• organ movement,
• image registration artifacts,
• animal and study management,
• no anatomical CT priors/landmarks for OT/SPECT diagnosis,
• SPECT attenuation correction using CT data,
• individual tracer kinetics, and
• problem of subject encumbrance.

Some preferred applications of the triple-modality imaging system according to the invention are to detect and state tumors, to image specific cellular and molecular processes (e.g. gene expression, or more complex molecular interactions such as protein-protein interactions), to monitor multiple molecular events simultaneously, to track single or dual-labelled cells, to optimize drug and gene therapy, to image drug effects at a molecular and cellular level, to assess disease progression at a molecular pathological level, especially to create the possibility of achieving all of the above goals of imaging in a single, rapid, reproducible, and quantitative manner.

Further uses of the present invention comprise monitoring time-dependent experimental, developmental, environmental and therapeutic influences on gene products in the same animal (or patient), studying the interaction of tumor cells and the immune system, studying viral infections by marking the virus of interest with a reporter gene, and many others. There is also an enormous clinical potential for the non-invasive assessment of endogenous and exogenous gene expression in vivo (gene (DNA), message (RNA), protein, function), for imaging receptors, enzymes, transporters, for novel applications in basic and translational research (gene therapy, etc.), for early detection of disease, for guidance for therapeutic choices, for monitoring drug action, for aid of pre-clinical drug development, for non-invasive and repetitive monitoring of gene therapy, and for optimizing clinical trials of human gene therapy.

An optical imaging detector in the context of the present invention is a device capable of acquiring images of at least part of an imaged object by detecting fluorescent or bioluminescent signals (i.e. light) emitted from the imaged object. The imaged object can be any object known by those skilled in the art, which is accessible by optical imaging. Preferably the imaged object is an intact living organism like a small animal or sections of a human being such as breast or head. Preferably, the optical imaging detector comprises at least on CCD camera.

In a preferred embodiment of the present invention, the SPECT detector comprises at least one (e.g. planar) scintillation crystal array with a multiplicity of scintillation crystals and at least one spatially resolving photo-detector. The scintillation crystals are dense, transparent crystalline materials (for example NaI(T1)) that serve as converters for high-energy gamma-rays into visible light. The visible light is detected in the form of electrical signals in spatially resolving fashion by the photo-detector.

In a preferred embodiment of the present invention, the x-ray source is a x-ray tube with an anode voltage of up to 100 kV (e.g. 50 kV) at up to 100 watts (e.g. 50 watts), maximized for a small focal spot size of less than 50 microns (e.g. 35 microns), and a cone angle in the range of 10 to 60 degrees (e.g. 24 degrees). The x-ray detector is a high-resolution planar camera with a sensitive size of up to 20cm x 20 cm, depending of subject size (e.g. 5cm x 10 cm). The image sensor contains a pixilated photo sensor (e.g. 1024x2048 pixel photodiode array (e.g. CMOS, CCD)) which is mounted on a phosphor screen (e.g. a Gd₂O₂S scintillator) which converts the incident x-rays to visible light.

According to preferred embodiments of the present invention the optical imaging detector, the SPECT detector, and the x-ray source and detector are mounted on a common rotatable gantry, the gantry being designed like an optical bench. Preferably the optical imaging detector, the SPECT detector, the x-ray detector, the x-ray source and the light source are modules, which are spatially rearrangeable with respect to one another. By designing the common rotatable gantry like an optical bench, the detectors, collimators, lasers, x-ray sources and any other mechanical or optical elements can be mounted ad libitum for an optimal triple-modality application specific imaging system setup. A modular concept is particularly advantageous because the performance characteristics of the diverse imaging detectors might change under various exams, combinations, and requirements, and should therefore be adaptable and optimizable. Exemplarily, both gamma- and optical cameras can be aligned either to image the object from different projection angles or to do so from identical projection angles, depending on study design. The latter case might be unavoidable or desired when the study at hand is planar imaging. The former case, on the other hand, might be favourable in terms of optimal spatial resolution and sensitivity setup. The proposed integration concept is laid out in a fully modular manner such that a wide range of applications can be performed where only anatomical or only functional and/or molecular information is required, i.e. any of these exams can be performed independently. Such design yields highest possible sub-modality performance while minimizing radiation dosage.

According to preferred embodiments of the triple-modality imaging system according to the invention the imaged object is placed at the rotation axis of the gantry, the fields-of view of the optical imaging detector, the SPECT detector and the x-ray detector over-lapping spatially at the rotation axis. The method according to the invention preferably comprises rotating the optical imaging detector, the SPECT detector, and the x-ray source and detector on a common rotatable gantry around a rotation axis, the imaged object being located at the rotation axis. According to these embodiments the fully integrated OT, SPECT, and CT modalities employ specific detectors which are mounted on a common gantry whereby projection images are acquired around a single axis of rotation with axially unshifted (i.e. identical) spatially over-lapping fields-of-view (FOV) of the involved detectors.

Preferably, the optical imaging detector, the SPECT detector, the x-ray source and detector, and the imaged object of the triple-modality imaging systems are arranged within a light-tight and x-ray shielded compartment. For fluorochrome excitation, the triple-modality imaging system can comprise at least one light source (e.g. laser) arranged to illuminate at least part of the imaged object to facilitate fluorescence imaging. The light source is preferably also mounted on the common rotatable gantry, together with the optical imaging detector, the SPECT detector, the x-ray source and the x-ray detector

For example, in fluorescence imaging the at least one light source illuminates at least part of the imaged object with light of an excitation wavelength in order to excite fluorescence probes within the imaged object, resulting in the stimulated emission of light with a shifted wavelength. Fluorescence mediated optical imaging requires a light source preferably of variable selectable wavelengths to be integrated into the imaging system for fluorochrome excitation. The optical imaging detector of the triple-modality imaging system according to the invention can further comprise at least one filter in the optical transmission pathway from the imaged object to the optical imaging detector for filtering out light of the at least one light source. Such a filter can be provided e.g. for the purpose of filtering out excitation light, when the detector is used for fluorescence imaging.

For bioluminescence imaging no filter is needed. The filter is preferably removable or replaceable. Different filters can be used for different optical probes/markers needing excitation light of a specific wavelength which requires appropriate filter arrangements.

In one embodiment of the present invention the triple-modality imaging system comprises a layer in front of the SPECT detector which essentially reflects photons of bioluminescent and/or fluorescent markers and which essentially transmits photons of radioactive markers. This embodiment is favourably used for simultaneous imaging of both photon types from a single projection angle. The photons of the bioluminescent and/or fluorescent markers having a first average energy and the photons of the radioactive markers having a second average energy can be separated for the separate detection with the aid of the layer. The layer essentially reflects or transmits the photons in a manner dependant on the energy. By way of the example, gamma-radiation is transmitted without or with only slight interactions with the layer, while the lower-energy optical radiation is reflected by the layer. This enables separate detection of the photons having different energies which are admitted at the same projection angle by markers in the object to the image. Furthermore, the layer can serve for reflecting the photons of the bioluminescent and/or fluorescent markers in the direction of the optical imaging detector and for transmitting said photons of the radioactive markers in the direction of the SPECT detector. Consequently, the photons having different energies are not only separated by the layer, but are also already deflected into the direction of the optical detector which detects them.

The present invention also refers to a triple-modality imaging method including the steps of reconstructing an optical image, a SPECT-image, and an x-ray image by the acquired optical imaging data, SPECT-data, and x-ray data, and displaying at least one of the optical image, the SPECT-image, the x-ray image, a fused SPECT/optical image, a fused SPECT/x-ray image, a fused x-ray/optical image, or a fused optical/SPECT/x-ray image on a display device.

The present invention is explained in greater detail below with reference to preferred embodiments described in an example and shown in the drawing.

### Example

According to a preferred embodiment of the triple-modality imaging system according to the present invention three imaging detector sub-systems (OT/SPECT/CT) are mounted on a common rotatable and translatable gantry whereby the entire assembly is mounted in a light-tight and gamma-ray shielded compartment. This compartment is mounted on a movable trolley which contains another compartment holding all necessary camera control, data read-out, laser light, gantry and linear stage motion control electronics as well as high-voltage and power supply, and workstation computers.

The key design features of the SPECT gamma camera sub-system are a 2x2 array of Hamamatsu H8500 position sensitive photomultiplier tubes (PSPMTs) and a pixellated scintillator array (St. Gobain Crystals and Detectors). The scintillation crystal consists of a 66x66 array of individual 1.3 x 1.3 x 6 mm³ NaI(Tl) crystal elements that are separated by a 0.2 mm thick layer of white reflective epoxy, for a crystal pitch of 1.5 mm and a total array size of 9.9 x 9.9 cm². The SPECT detector head is housed within a tungsten body providing a mounting for a changeable collimator frame. Each H8500 PSPMT has an 8x8 array of anode pads where the gains of the anode pads were balanced by individual resistors. The voltages from the anode pads of the four PSPMTs are summed in each direction to provide 16 x- and 16 y-signals, which are used for energy determination and for a truncated center of gravity calculation for event positioning. A raw image from uniform irradiation with 511 keV photons is used for crystal location determination. The gamma ray energies then were normalized individually for each crystal. The real-time data acquisition control system is written using Kmax (Sparrow Corp.). The control computer is a Dell Optiplex GX280 with a 3.6 GHz Pentium 4 processor. Depending on the user-preferred setup, various collimators (pinhole, parallel beam, fan beam, cone beam, or others) can be attached to the gamma-camera housing. Exemplarily, a custom-made pinhole collimator suitable for attachment of reflective surfaces for a specific mode of optical imaging suitable for attachment of reflective surfaces for a specific mode of optical imaging has been built. Various pinhole inserts are produced made of tungsten alloy (Densimet, Plansee AG, Reutte, Austria). The focal length of this collimator is 7.5 cm.

The high imaging resolution ORCA AG cooled CCD camera (Hamamatsu) is used for the optical imaging detector sub-system. This digital camera contains a progressive scan interline CCD chip featuring 1.37 million pixels and generates 12 bit digital output at very high quantum efficiency and low noise which makes it suitable for low light level imaging. A near infrared wavelength corrected objective (CNG compact, Jos. Schneider, Bad Kreuznach, Germany) is attached to the camera in this example. A high performance serial bus IEEE 1394 is used to transmit the output to another control computer. Various diode laser sources (LG Laser Technologies, Kleinostheim, Germany, or others) are available for changeable wavelength fluorochrome excitation. To filter out fluorescence light at the detector two long-pass filters are mounted in this assessment in front of the objective (715 nm and 695 nm Schott glass filters, B+W Filer, Bad Kreuznach, Germany).

The x-ray CT sub-system consists of an x-ray tube, power supply, a detector, and a PC PCl digital frame grabber board. For the x-ray tube, a series 5000 Apogee (Oxford Instruments X-Ray Technology, Inc., Scoots Valley, CA) is used here. This tube has a maximum power of 50 W, at 4 to 50 kV, 0 to 1 mA. The focal spot size is 35 µm and the cone angle is 24 degrees. A high-voltage power supply (HXR-505-50-01, Matsusada Precision Inc., Japan) delivers 0 to 50 kV, 0 to 1 mA output voltage for the x-ray tube. The x-ray detector is a Shad-o-Box 2048 (Rad-icon Imaging Corp. Santa Clara, CA). This x-ray camera is a complete detection system for high-resolution radiation imaging. The heart of the Shad-o-Box camera is a two-dimensional photodiode array containing 1024 by 2048 pixels on 48 µm centers. A Gd202S scintillator screen, placed in direct contact with the photodiode array, converts incident x-ray photons to light, which in turn is detected by the photodiodes. A carbon-fiber window shields against ambient light and protects the sensitive electronics from accidental damage. The analog signal from the photodiode sensor is digitized to 12-bit resolution in four parallel A/D channels, and then interleaved for maximum transmission speed across a high-speed parallel digital interface. This interface consists of a 68-pin mini-D (SCSI-3) receptable and conforms to the AIA (Automated Imaging Association) A15.08 specification. Pixel clock, line enable and frame enable signals are available at the connector to facilitate acquiring the image data with a standard digital frame grabber. The Shad-o-Box 2048 camera delivers 4000:1 dynamic range (defined as the maximum signal divided by the read noise) at a maximum frame rate of 2.7 frames per second.

The most innovative aspect of the triple-modality (SPECT-OT-CT) small animal imaging design proposed in the example, is that it is possible to perform unified simultaneous acquisition, reconstruction, attenuation and scatter correction, tracer/probekinetic modeling, and fused planar or tomographic image display.

Since both regional distribution and time variation of the underlying multi-variate optical and isotopic photon emission distributions as well as x-ray transmission maps are acquisition and subject specific and diversified by variations thereof, and imaging procedures cannot be performed repeatedly at short time intervals on the same living object in many cases, combined and simultaneous imaging is desired and possible with this novel device carrying clearly advantageous potential. Further advantages are simultaneous recording of tracer kinetics, imaging of multiple distinctive molecular processes as part of an investigated molecular pathway, less subject encumbrance, and identical imaging geometries. The proposed triple-modality tomographic imaging system has the potential to accurately quantify fluorescence, bioluminescence, and radiopharmaca distributions in deep heterogeneous media in vivo at high spatial resolution and correlation to the anatomy of the imaged object provided by x-ray.

The invention supports the development of generalized reporter probes and x-ray contrast. The CT scan can be used in many exams to improve the SPECT (and possibly OT) scan to become more quantitatively accurate. Also, CT data offer better options for attenuation correction as a SPECT transmission scan would provide, even for small animals, since SPECT transmission scans suffer from poor image quality, in part because of the low radiation flux of isotopic sources such as gadolinium-153 as compared to x-ray tube generated radiation flux.

Of all, single-step simultaneously acquired molecular, functional, and anatomical information, a common projection data acquisition and image reconstruction protocol, as well as its fused representation are the most noticeable and important advantages of this invention for the potential user.

### Description of the Figures

- Fig. 1: shows a triple-modality imaging systems according to the invention, and
- Fig. 2: shows a triple-modality imaging system according to Fig. 1, further showing the field-of-view of each detector.

### Detailed Description of the Figures

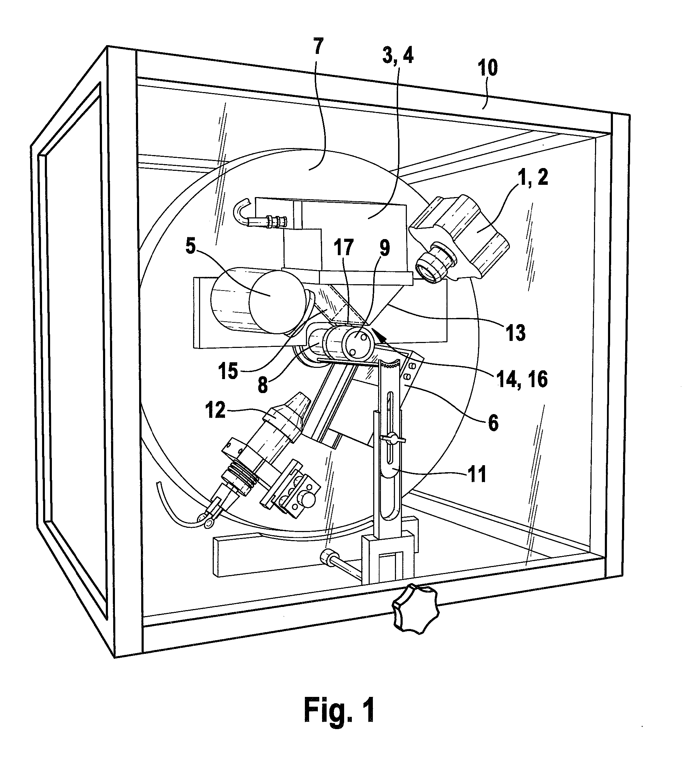

Fig. 1 shows a triple-modality imaging systems according to the invention.

The triple-modality imaging system comprises an optical imaging detector 1, which is a CCD-camera 2, for acquiring optical imaging data of an imaged object. It also comprises a SPECT detector 3, which includes a gamma-camera 4 for acquiring SPECT data of the imaged object. Furthermore, the systems comprises an x-ray source 5 (an x-ray tube) and an x-ray detector 6 for acquiring x-ray data of the imaged object. All of these components of the triple-modality imaging system are mounted on a common rotatable gantry 7, which is designed like a circular optical bench. The optical imaging detector 1, the SPECT detector 3, the x-ray detector 6, and the x-ray source 5 are modules, which are spatially re-arrangeable on the gantry 7, on which they are releaseably mounted. The gantry 7 is rotatable around a rotation axis 8. A receptacle 9 (object holder) is mounted concentrical of the rotation axis 8. The receptacle 9 is provided for receiving the imaged object, e.g. a mouse or a phantom that can be used to assess the physical properties of the imager. It does not rotate with the rotatable gantry 7, but is non-rotatably fixed via a holder 11 to the inner wall of a light-tight and gamma-ray shielded compartment 10, which surrounds the gantry 7 with the detectors 1, 3, 6.

The optical imaging detector 1, the SPECT detector 3, the x-ray source 5, and the x-ray detector 6 are all aimed at the rotation axis 8 and, thus, at the receptacle 9 for the imaged object, the fields-of-view of the optical imaging detector 1, the SPECT detector 3, and the x-ray detector 6 over-lapping spatially at the rotation axis 8.

The system further comprises a light source 12, which is a laser, for illuminating an imaged object within receptacle 9. The light source 12 is mounted on the common rotatable gantry 7. The light source 12 is also a module, which is spatially re-arrangeable on the gantry 7, on which it is releaseably mounted.

A shielding 13 is arranged in front of the SPECT detector 3. The shielding 13 is bent at a bending edge 14 at an angle of 90°. A layer 15 is fixed on the shielding 13, which essentially reflects photons of bioluminescent and/or fluorescent markers, and which essentially transmits photons of radioactive markers. The layer 15 is likewise bent at a bending edge 16 at an angle of 90°. The bending edge 16 of the layer 15 lies on the bending edge 14 of the shielding 13. The layer 15 covers an aperture 17 of the shielding 13. The shielding 13 with the aperture 17 serves as a collimator for the SPECT detector 3. The layer 15 reflects optical photons, e.g. photons from bioluminescent and/or fluorescent markers, and transmits γ-photons from radioactive markers.

Preferably, the radioactive markers comprise at least one marker from the group As-72, Br-75, Co-55, Cu-61, Cu-67, Ga-67, Gd-153, I-123, I-125, I-131, In-111, Ru-97, T1-201, Tc-99m and Xe-133. The radioisotope respectively used is selected as a marker with regard to its half-life and the energy of the radiation that it emits, in a manner dependent on the biological process to be measured.

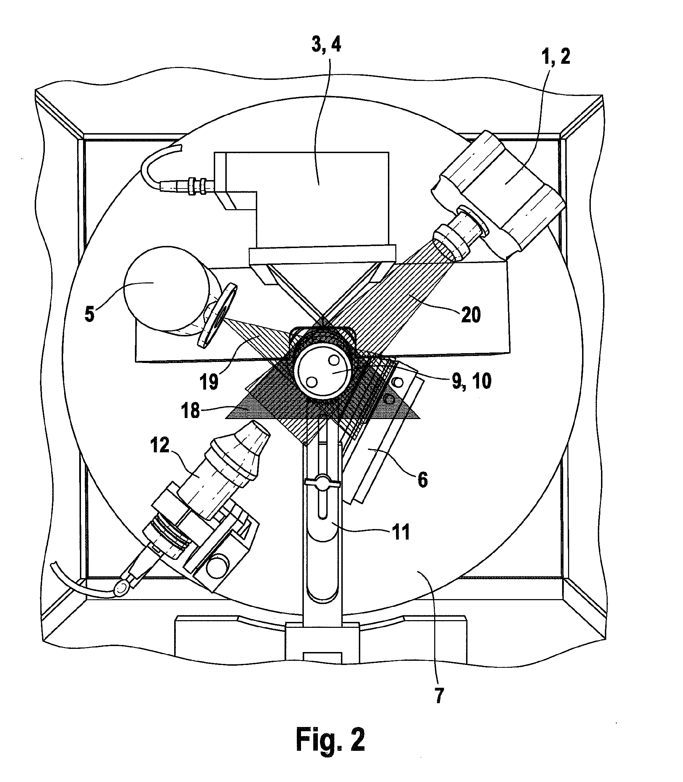

Fig. 2 shows the same triple-modality imaging system as Fig. 1 with similar reference Nos. referring to similar parts. Additionally, the SPECT field-of-view 18, the CT field-of-view 19, and the OT field-of-view 20 are depicted. The optical imaging detector 1, the SPECT detector 3, and the x-ray detector 6 have spatially over-lapping fields-of view 20, 18 and 19. They are arranged to acquire optical imaging data, SPECT data and x-ray data of an imaged object within the receptacle 9 similtaneously.

### Reference Numbers

- 1: optical imaging detector
- 2: CCD camera
- 3: SPECT detector
- 4: gamma camera
- 5: x-ray source
- 6: x-ray detector
- 7: gantry
- 8: rotation axis
- 9: receptacle
- 10: compartment
- 11: holder
- 12: light source
- 13: shielding
- 14: bending edge of shielding
- 15: layer
- 16: bending edge of layer
- 17: aperture
- 18: SPECT field-of-view
- 19: CT field-of view
- 20: OT field-of view

## Claims

1. A triple-modality imaging system, wherein an optical imaging detector (1) for acquiring optical imaging data, a single photon emission computed tomography (SPECT) detector (3) for acquiring SPECT-data, and an x-ray source (5) and detector (6) for acquiring x-ray data are arranged to acquire the optical imaging data, the SPECT data, and the x-ray data of an imaged object simultaneously, the optical imaging detector (1), the SPECT detector (3), and the x-ray detector (6) having spatially over-lapping fields-of-view (FOV) (20, 18, 19).

2. The triple-modality imaging system according to claim 1, wherein the optical imaging detector (1), the SPECT detector (3), and the x-ray source (5) and detector (6) are mounted on a common rotatable gantry (7), the gantry (7) being designed like an optical bench.

3. The triple-modality imaging system according to claim 2, wherein the imaged object is placed at a rotation axis (8) of the gantry (7), the fields-of-view (20, 18, 19) of the optical imaging detector (1), the SPECT detector (3), and the x-ray detector (6) over-lapping spatially at the rotation axis (8).

4. The triple-modality imaging system according to one of claims 1 to 3, wherein the optical imaging detector (1), the SPECT detector (3), the x-ray source (5) and detector (6), and the imaged object are arranged within a light-tight and gamma-ray shielded compartment (10).

5. The triple-modality imaging system according to one of claims 1 to 4, further comprising a light source (12) for illuminating the imaged object.

6. The triple-modality imaging system according to one of claims 1 to 5, wherein the optical imaging detector (1), the SPECT detector (3), the x-ray detector (6), and the x-ray source (5) are modules, which are spatially rearrangeable with respect to one another.

7. The triple-modality imaging system according to one of claims 1 to 6, comprising a layer (15) in front of the SPECT detector (3), which essentially reflects photons of bioluminescent and/or fluorescent markers, and which essentially transmits photons of radioactive markers.

8. A triple-modality imaging method comprising simultaneously acquiring optical imaging data with an optical imaging detector, single photon emission computed tomography (SPECT) data with a SPECT detector and x-ray data with an x-ray detector of an imaged object, which is placed within spatially over-lapping fields-of view (FOV) of the optical imaging detector, the SPECT detector and the x-ray detector.

9. The method according to claim 8, comprising rotating the optical imaging detector, the SPECT detector, and the x-ray detector on a common rotatable gantry around a rotation axis, the imaged object being located at the rotation axis.

10. The method according to one of claims 8 or 9, including the steps of reconstructing optical images, SPECT images and x-ray images by the acquired optical imaging data, SPECT data, and x-ray data, and displaying at least one of the optical images, the SPECT images, the x-ray images, a fused SPECT/optical image, a fused SPECT/x-ray image, a fused x-ray/optical image or a fused optical/SPECT/x-ray image on a display device.
